(19) Europäisches Patentamt / European Patent Office / Office européen des brevets

(11) **EP 4 458 402 A2**

(12) **EUROPEAN PATENT APPLICATION**

(43) Date of publication:
 **06.11.2024 Bulletin 2024/45**

(21) Application number: **24195347.0**

(22) Date of filing: **12.05.2021**

(51) International Patent Classification (IPC):
 **A61M 25/01** *(2006.01)*

(52) Cooperative Patent Classification (CPC):
 **A61B 1/0052; A61B 1/00128; A61M 25/01;**
 A61M 2025/0166

(84) Designated Contracting States:
 **AL AT BE BG CH CY CZ DE DK EE ES FI FR GB
 GR HR HU IE IS IT LI LT LU LV MC MK MT NL NO
 PL PT RO RS SE SI SK SM TR**

(30) Priority: **12.05.2020 DK PA202070309**

(62) Document number(s) of the earlier application(s) in
 accordance with Art. 76 EPC:
 **21726325.0 / 4 149 339**

(71) Applicant: **Coloplast A/S
 3050 Humlebaek (DK)**

(72) Inventor: **SCHERTIGER, Lars Olav
 3480 Fredensborg (DK)**

Remarks:
 This application was filed on 20-08-2024 as a
 divisional application to the application mentioned
 under INID code 62.

(54) **ACCESSORY DEVICE FOR A CATHETER**

(57) An accessory device for assisting insertion of a catheter is disclosed. The accessory device comprises a handle comprising an attachment means for releasably coupling a catheter to the handle, and a camera for capturing one or more images, and a monitor in communication with the camera is disclosed. The monitor comprises a display configured to display the one or more images. The accessory device provides for visualizing the meatal area on a monitor by means of a camera moving in unison with the catheter, such that the user is assisted in identifying the meatus and inserting the catheter.

Fig. 1

EP 4 458 402 A2

## Description

The present disclosure relates to an accessory device for assisting insertion of a catheter and a catheter system comprising such an accessory device and a catheter.

## Brief description of the drawings

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.

Fig. 1 illustrates a perspective view of an accessory device according to an embodiment of the invention,

Fig. 2 illustrates a perspective view of a handle of an accessory device according to an embodiment of the invention,

Fig. 3 illustrates a perspective view of a handle of an accessory device according to an embodiment of the invention,

Fig. 4A illustrates a perspective view of a handle of an accessory device according to an embodiment of the invention,

Fig. 4B illustrates a trigonometric representation between a first optical axis and a first catheter axis as defined by structural features of a handle of an accessory device according to an embodiment of the invention,

Fig. 5 illustrates a short and a long insertable portion of a catheter coupled to a handle of an accessory device according to an embodiment of the invention,

Fig. 6A illustrates a perspective view of a monitor of an accessory device,

Fig. 6B illustrates a perspective view of a monitor of an accessory device,

Fig. 7 illustrates a method of using an accessory device, and

Fig. 8 illustrates a schematic view of an accessory device according to an embodiment of the invention.

## Detailed description

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

The present disclosure relates to an accessory device for assisting insertion of a catheter. In particular, the accessory device is suitable for use with intermittent urinary catheters where an increased focus is on avoiding urinary tract infections (UTIs). Therefore, a need exists to enable easy insertion of a catheter without contaminating the catheter during the catherization procedure.

Urinary catheter assemblies for draining the bladder are increasingly used for intermittent as well as indwelling or permanent catheterization. Typically, urinary catheters are used by patients suffering from urinary incontinence or by disabled individuals like paraplegics or tetraplegics, who may have no control permitting voluntary urination and for whom catheterization may be the way of urinating.

Urinary catheters are divided into two major groups of catheters, indwelling catheters and intermittent catheters. Indwelling catheters are typically inserted into the urethra and the bladder by medical personal (i.e. a trained professional, typically a nurse or physician) and has means for retaining the catheter inside the bladder for up to two weeks or more. Indwelling catheters are soft and flexible since they have to remain in the urethra for weeks. Indwelling catheters empty the bladder continuously.

Intermittent catheters are typically inserted by the user him- or herself and sits only in the urethra and bladder for as long as it takes to empty the bladder - e.g., for about 5-10 minutes. Intermittent catheters are used every 4-6 hours to empty the bladder corresponding roughly to the interval that people having no urinary problems will usually go to the bathroom. Intermittent catheters are typically more rigid than indwelling catheters since they have to be inserted by the user him-/herself and since they do not need to sit in the urethra for days or weeks. An important feature for the intermittent catheter is to ease the insertion into the urethra. This is done by providing the intermittent catheter with a low frictious surface. Non-limiting examples of such are hydrophilic coated catheters which are subsequently wetted by a swelling media in order to produce a low friction surface, or oil or water-based gel which is applied to the catheter before insertion into the urethra.

Intermittent urinary catheters may be provided with a hydrophilic coating that needs to be wetted prior to use and thereby absorbs a considerable amount of liquid. Such a hydrophilic coating will provide a very lubricious surface that has very low friction when the catheter is to be inserted. Hydrophilic coated catheters, where the coating absorbs a considerable amount of liquid for a low frictious surface (swelling degree >100%), will not be suitable for indwelling catheters, because the hydrophilic surface coating would stick inside the mucosa of the urethra if left inside the body for a longer period, due to the hydrophilic coating transforming from being highly lubricious when fully wetted to being adhesive when the hydration level of the coating is reduced.

The accessory device as disclosed herein is particularly suitable for use with intermittent catheters to be inserted by the user him- or herself.

In the following, whenever referring to a proximal end of an element, the referral is to the end adapted for insertion. Whenever referring to the distal end of an element, the referral is to the end opposite the insertion end. In other words, the proximal end is the end closest to the user, when the catheter is to be inserted and the distal end is the opposite end - the end furthest away from the user when the catheter is to be inserted.

The longitudinal direction is the direction from the distal to the proximal end. The transverse direction is the direction perpendicular to the longitudinal direction, which corresponds to the direction across the shaft of the catheter.

In the following, whenever referring to a device being "releasably couplable" to another device, it should be understood that the devices may be temporarily attached in such a way that the risk of unintentionally separating them is minimal. However, the devices should be attached in such a way that they may be separated if desired.

A catheter, in particular an intermittent urinary catheter, suitable for use with an accessory device according to embodiments disclosed herein comprises a main tubular part extending from the distal end to the proximal end. The tubular portion can be cylindrical or conical. In embodiments, the tubular portion has an oval cross-section. The tubular portion is configured for providing urine flow through the intermittent catheter from a drainage portion to the distal end. A closed tip portion with a closed tip is positioned in the proximal end of the catheter and is provided as a rounded closed end of the tube constituting the main tubular portion of the catheter. The drainage portion of the tubular portion will typically be in the proximal portion of the tubular portion. In embodiments, the drainage portion includes multiple drainage openings providing for flow of urine between the outside of the catheter and an inside lumen of the tubular portion. The catheter may comprise a connector in the distal end and may in an embodiment comprise a flared end of the catheter so that the diameter of the connector increases with respect to the tubular part. The connector may function as a handle allowing the user to manipulate the catheter, and/or the catheter may comprise a handle in the distal end, which has a length allowing the user to manipulate the catheter. The catheter comprises an insertable portion proximal to the connector and/or handle, the insertable portion extending from the tip in the proximal end to the connector.

Usually, catheters used as urinary draining devices are from size 8 FR to size 18 FR. FR (or French size or Charriere (Ch)) is a standard gauge for catheters approximately corresponding to the outer circumference in mm. More accurately, the outer diameter of the catheter in mm corresponds to FR divided by 3. Thus 8 FR corresponds to a catheter with an outer diameter of 2.7 mm and 18 FR corresponds to a catheter with an outer diameter of 6 mm. Catheters for use with an accessory device of the present disclosure may prior to use be provided with a hydrophilic coating so as to impart a low-friction insertion.

Catheters particularly suitable for use with an accessory device according to the present disclosure may be female intermittent urinary catheters comprising an insertable portion having a length between 55 mm and 200 mm. For example, a category of female intermittent urinary catheters may be compact catheters having an insertable portion having a length between 55 mm and 80 mm, another category may be straight catheters having an insertable portion having a length between 140 and 200 mm, and an intermediate category may be catheters having an insertable portion having a length between 80 mm and 140 mm.

Whereas the above discussion relates to urinary catheters, and in particular intermittent urinary catheters, it is envisioned that the accessory devices as described herein may be used with other types of catheters as well, in particular catheters to be inserted by the user him- or herself, such as anal catheters for use with bowel irrigation systems, or wherever a catheter may need to be inserted, in particular where ever the meatus is difficult to identify for different reasons.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do not provide the effect.

The present disclosure provides an accessory device for assisting insertion of a catheter and a catheter system for assisting insertion of a catheter.

In a first aspect, an accessory device for assisting insertion of a catheter is disclosed. The accessory device comprises a handle comprising an attachment means for releasably coupling, such as receiving and releasably coupling, a catheter to the handle, and a camera for capturing one or more images, and a monitor in communication with the camera. The monitor comprises a display configured to display the one or more images.

For some, the insertion of a catheter, such as a urinary intermittent catheter, can be difficult for different reasons, including difficulties in seeing and/or identifying the meatus in the meatal area, e.g., due to the presence of skin folds, excessive skin, etc. Further, to avoid urinary tract infections (UTI's), it is advised to insert the catheter without contaminating the sterile insertable portion by touching the surrounding meatal area. The issues described herein are particularly pronounced for women, but the accessory device may be used with catheters suitable for both men and women.

Present embodiments provide a handheld accessory device for visualizing the meatal area on a monitor by means of a camera moving in unison with the catheter, such that the user is assisted in identifying the meatus and inserting the catheter. In particular, an easy-to-use, handheld and portable accessory device allowing the user to bring the device with him/her and use it during each catherization is provided.

Embodiments provide an accessory device having a handle configured to receive a catheter, such that a catheter can be coupled/attached to the handle. In particular, an attachment means of the handle is configured to releasably couple a catheter to the handle. For example, the attachment means may receive a connector of the catheter. Further, the handle comprises a camera for capturing one or more images, preferably of the proximal end of a catheter coupled to the handle and the surroundings thereof (e.g., the meatal area when the catheter and handle are brought into proximity thereof). In embodiments, the camera is substantially longitudinal (e.g., along an optical axis) and may have a diameter (e.g., of a lens of the camera) of less than 15 mm, such as less than 10 mm, such as in the range from 3 mm to 8 mm, in order to provide a compact handle. The camera may be any commercially available camera suitable for the intended use. In embodiments, the camera comprises an image sensor, such as a CCD sensor or a CMOS sensor.

In a preferred embodiment, the camera is configured to capture a plurality of images, such as to form a video. In embodiments, the plurality of images is captured (by the camera) and displayed (on the display) consecutively to form a video (e.g., with a frame rate of at least 20 FPS). Further, preferably, the video is transmitted/broadcasted to the display of the monitor, without delay, in order to provide a live video footage of the objects being within the field of view of the camera. Thereby, the movements as caused by the user handling the handle are reflected in the images/video displayed in the display. Thereby, the user can control/manipulate the catheter by holding on to the handle and moving the handle in accordance with what he/she sees on the monitor. Thereby, the user may identify meatus on the display and easily insert the catheter according to the images/video of the monitor. In particular, the user may guide the tip of the catheter into the meatus by observing the tip of the catheter on the display. When the user, e.g., guided by the displayed images/video, has inserted the catheter into the meatus, he/she may keep the handle coupled to the catheter during micturition/voiding, or he/she may decouple the catheter from the handle and proceed with the micturition/voiding.

In embodiments, the handle has a substantially rectangular cross section having side lengths not exceeding 50 mm, such as to provide a small handheld device. In embodiments, the handle is made of a plastic material and/or a metal, such as aluminium. In embodiments, the handle is formed in an injection moulding process or a 3D printing process. In embodiments, the handle comprises an organically shaped body, such as to provide an ergonomic grip.

In embodiments, the monitor comprises a housing having a front comprising the display, and a back. In embodiments, the housing is made in an (injection) moulding process or a 3D printing process. In embodiments, the monitor comprises a power unit, such as a battery, for powering the display. In embodiments, the camera, and optionally a light source, is powered by the power unit of the monitor. In embodiments, the handle comprises a power unit, such as a battery, for powering the camera. In embodiments, the monitor comprises a processor for processing the images as captured by the camera and for displaying the images on the display. In embodiment, the monitor comprises a memory, such as a volatile memory, such as random-access memory (RAM). In embodiments, the monitor comprises a power button for turning on the monitor (and display thereof) and/or the camera. In embodiments, the power button turns on the monitor and the camera, such that the accessory device is ready for use in a single action. In embodiments, the monitor and/or handle comprises a charging port connected to a battery, such as a waterproof or watertight charging port. In embodiments, the charging port is of a USB-type. In embodiments, the charging port is connected to the power unit and the processor, such as for providing for updating a firmware of the processor. In embodiments, the monitor and/or handle comprises means for wireless charging, such that the monitor and/or handle may be wirelessly charged, such as to reduce the risk of water affecting the functionality of the accessory device.

In embodiments, the monitor, such as the processor thereof, is configured to mirror the one or more images in the display. Thereby, movements of the handle (e.g., caused by the user) based on the one or more images are intuitively displayed in the display. In other words, by mirroring the images in the display, movements of the handle in a certain direction are reflected by a movement in the same certain direction in the display. Thereby, the user may more easily construe the movements he/she causes the handle and catheter coupled thereto.

In embodiments, the display is a commonly available display suitable for the disclosed use. For example, the display may be an LCD display or an LED display. In embodiments, the display is between 1" and 5" in size (inches in diagonal), such as to provide a small monitor, which is easy to carry. In embodiments, the display has a size of 1", or 2", or 3", or 4", or 5", or more than 5". In embodiments, the display is touch-sensitive, such as to provide for detecting touch gestures, such as pinching to zoom on the images or to turn on/off a light source.

In an embodiment, the accessory device has a liquid ingress protection rating of at least IPX4 according to the IEC standard 60529. For example, the accessory device may have a liquid ingress protection rating of IPX4, or of IPX5, or of IPX6, and/or of IPX7, and/or of IPX8. Increasing the rating allows for an increased versatile use of the accessory device, such as providing for an accessory device being washable, such as to clean the device in liquids/water after use. Further, the liquid ingress protection rating provides for protecting the accessory device during the catherization procedure, where exposure to liquids, such as urine, is a possible outcome. In embodiments, at least the handle of the accessory device has a liquid ingress protection rating according to the above embodiment.

In an embodiment, the attachment means comprises a clip for releasably coupling a connector of a catheter to the handle. Thereby, the insertable portion of the catheter remains sterile and is not confined by the attachment means or the handle as such, such that the insertable portion extends into free space, along a first catheter axis, as will be discussed below. Further, since the insertable portion is commonly provided with a coating providing a low friction, coupling only the connector to the handle provides for easy attachment - the user can hold on to the connector and couple it to the handle without touching the sterile insertable portion.

In embodiments, the clip is configured to receive a cylindrical or conical portion, such as a cylindrical or conical portion of connector, having a diameter less than 15 mm, such as in the range from 5 mm to 15 mm, such as between 8 mm and 13 mm.

In embodiments, the clip comprises one or more flexible elements, such as an element supported by a spring and/or elastic material, such that the catheter can be coupled to the clip by manipulating/actuating the flexible element. In embodiments, the flexible element is made of a plastic material, such as a rigid plastic material supported by a spring, and/or a rubber material.

In an embodiment, the clip is configured for fixating a connector of a catheter by means of friction. In an embodiment, the clip comprises a conical inner shape configured to receive and fixate a connector of a catheter in a sliding motion.

In embodiments, the clip is a rigid (e.g., made of a plastic material) cylindrical portion or conical portion (or a part thereof) having an inner curvature corresponding to an outer curvature of a catheter, such as a connector thereof, suited for the given inner curvature of the clip. Thereby, it may receive a catheter in a sliding motion, wherein the user slides the catheter, such as the connector thereof, into the clip. Thus, the clip according to the embodiment provides for fixating the catheter by means of friction. Likewise, the user may remove the catheter by sliding the catheter in an opposite direction. A conical inner shape provides for a gradually increasing friction between the connector and the clip as the user slides the connector into the clip. Thereby, the connector, and thus the catheter as such, becomes fixated in the clip in a way in which the catheter may still be removed by the user pulling it in the opposite direction by a force overcoming the friction. The conical inner shape may provide for using different types of catheters, provided they have a suitable connector: the increasing friction provides for sufficiently fixating different catheters to the clip.

In embodiments, the attachment means, such as in the form of a clip, is integral with the handle, such that the handle comprising the attachment means may be manufactured in a single (injection) moulding or 3D printing process. According to such manufacturing process, the camera may be installed subsequently, e.g., mounted (e.g., adhered) in a recess of the handle formed in the moulding or printing process.

In embodiments, the attachment means is manipulatable, such as tiltable, e.g., through a hinged portion, relative to the body of the handle and/or the camera, such that the user may tilt the attachment means relative to e.g., the camera. In embodiments, the tiltable attachments means is lockable relative to the body of the handle, such that, when the user has tilted the attachment means into a desired position, the user may lock the attachment means in such desired position. Thereby, the user may tilt the attachment means, such that a catheter coupled to the attachment means extend along a certain first catheter axis, as will be introduced below.

In embodiments, the camera is manipulatable, such as tiltable, e.g., through a hinged portion, relative to the body of the handle and/or the attachment means, such that the user may tilt the camera relative to e.g., the attachment means. In embodiments, the tiltable camera is lockable relative to the body of the handle, such that, when the user has tilted the camera into a desired position, the user may lock the camera in such desired position. Thereby, the user may tilt the camera, such that camera has a certain viewing direction relative to a certain first catheter axis of a catheter coupled to the attachment means, as will be introduced below.

By providing a manipulatable attachment means and/or camera, e.g., relative to each other, the user may adjust the accessory device according to the catheter he/she prefers to use, e.g., such that the camera points towards a desired object, e.g., tip of his/her preferred catheter. In alternative embodiments, as will be described in greater detail below, the camera and the attachment means are fixated relative to the handle and each other. However, according to such embodiments, as will be apparent from the following, a first optical axis of the camera may intersect a first catheter axis of the catheter to provide a versatile accessory device suitable for use with a range of different catheters without the need for adjusting the attachment means and the camera prior to use.

In an embodiment, the attachment means is configured for fixating a catheter along a first catheter axis. In embodiments, the attachment means defines a first catheter axis. In embodiments, the first catheter axis is coinciding with a longitudinal direction of a catheter, in particularly of an insertable portion thereof, coupled to the attachment means.

Thus, the first catheter axis is the axis along which a catheter coupled to the attachment means extends. In a preferred embodiment, the attachment means provides for fixating a catheter relative to the handle and/or the camera, such that said catheter is securely coupled to the handle, e.g., by means of the above-disclosed embodiments of the clip. Thereby, the catheter becomes fixated to the handle and will extend along a first catheter axis.

In an embodiment, the camera is fixated relative to the attachment means. By the camera and the attachment means being fixated relative to each other, such as in/on the handle, the user can manipulate a catheter coupled to the attachment means and see corresponding movements as captured by the camera on the monitor. In other words, by being fixated relative to each other, the camera and a catheter coupled to the attachment means move in unison when the user manipulates the handle to provide a steady image of the catheter and the meatal area.

In embodiments, the viewing direction of the camera points towards a tip of a catheter coupled to the attachment means, such that said tip is substantially in the centre of the images as captured by the camera. In embodiments, the tip is offset the centre of the image as captured by the camera, such as to provide a larger image of the meatal area on the display of the monitor.

In an embodiment, the camera comprises a first optical axis defining a viewing direction. Thus, camera may be said "to point" in a direction being a viewing direction defined by a first optical axis of the camera. In embodiments, the centre of a field of view of the camera is coinciding with the first optical axis. The first optical axis defines the viewing direction. Thus, objects being within the field of view, in particular in vicinity of the first optical axis, are captured in images by the camera.

In an embodiment, the first optical axis intersects the first catheter axis. Thereby, objects (e.g., a catheter), such as parts thereof (e.g., a tip of the catheter), arranged along the first catheter axis may be captured in images by the camera. The intersection may be created by appropriately engineering the handle, such that the attachment means provides for a coupled catheter being angled relative to the camera; in other words, that the first catheter axis is angled relative to the first optical axis by an angle providing an intersection between the axes. In embodiments, the angle is between 10 degrees and 30 degrees, such as between 15 and 25 degrees.

In an embodiment, the first optical axis intersects the first catheter axis at a distance between 55 mm and 200 mm from a lens of the camera. By measuring from a lens of the camera, alternatively from an image sensor of the camera, a fixed point of reference is provided. Alternatively, the distance may be measured from a proximal end of the attachment means, the proximal end being the part of the attachment means being closest to a tip of a coupled catheter. In embodiments, the proximal end of the attachment means may flush with a distal end of an insertable portion of the catheter, such that a distance from the proximal end of the attachment means to a tip of the catheter corresponds the length of the insertable portion of the catheter. In particular, by measuring from a lens of the camera, deviations caused by using different types of catheters with the accessory device is avoided. Whereas small deviations may occur from a trigonometric representation of the handle and corresponding axes, it is appreciated that providing the intersection at a certain distance from a lens of the camera, e.g., said certain distance corresponding to a length of the insertable portion of the catheter, will, in most circumstances, allow for the tip of the catheter to be within the field of view of the camera. Alternatively, the intersection as measured from a lens of the camera may be reduced by a factor depending on a distance between the camera and the attachment means of the handle and on the length of the insertable portion of the catheter coupled to the catheter. A trigonometric deviation supported by figures is provided in the detailed description of the figures.

By providing the intersection at a distance between 55 mm and 200 mm from a lens of the camera allows for using the accessory device with female intermittent urinary catheters commonly used in the field.

In an alternative embodiment, the first optical axis intersects the first catheter axis at a distance between 55 mm and 200 mm from any part of the handle, thereby providing a certain free space between the handle and the tip of the catheter and the meatal area.

By letting the first catheter axis and the first optical axis intersect, it is provided that a tip of the catheter is within the field of view of the camera. For example, where a user prefers using a catheter having an insertable length of 100 mm, the handle may be engineered to have the intersection at 100 mm from the camera lens, whereby it is ensured that the tip of the catheter is within the field of view of the camera.

In embodiments, the accessory device is optimised for a category of female intermittent catheters. For example, an accessory device suitable for use with compact catheters (insertable portion having a length of 55 mm to 80 mm) may have an intersection at a distance between 55 mm and 80 mm from a lens of the camera, such as at 67.5 mm. For example, an accessory device suitable for use with straight catheters (insertable portion having a length of 140 mm to 200 mm) may have an intersection at a distance between 140 mm and 200 mm from a lens of the camera, such as at 170 mm. For example, an accessory device suitable for use with intermediate catheters (insertable portion having a length of 80 mm to 140 mm) may have an intersection at a distance between 80 mm and 140 mm from a lens of the camera, such as at 110 mm.

In embodiments, the camera has a (e.g., fixed) focus distance corresponding to the distance from the lens of the camera to the intersection between the first optical axis and the first catheter axis, whereby focus distance is meant the distance from the camera lens to an optimal focus of the camera. Preferably, the optimal focus is at the tip of a catheter coupled to the handle, such that the focus distance corresponds to the distance from the camera lens to the tip of the catheter. Thereby, the tip of a catheter arranged in the intersection between the first optical axis and the first optical axis will be in focus in an image generated by the camera.

In embodiments, the camera comprises means for autofocussing, such as means for detecting and autofocussing on a tip of a catheter, thereby providing for focussing on a tip of different lengths of catheters.

In an embodiment, the camera has a depth of field of at least 60 mm. As commonly defined, the depth of field (DoF) is the distance between the nearest and the farthest objects that are in acceptably sharp focus in an image. A certain DoF may be provided by suitably engineering the optics of the camera (e.g., by choosing appropriate values for the focal length, aperture size and acceptable circle of confusion size, in conjunction with the distance to the object to be in focus).

A camera having a DoF of at least 60 mm provides for using a wide range of different catheters having insertable portions of different lengths and for having both the tip of the catheter and the vicinity thereof, such as the meatal area, in focus simultaneously. For example, a DoF of 60 mm provides for using catheters varying in length by up to 60 mm without having the tip of such a given catheter out of focus, provided that the above intersection between the first optical axis and the first catheter axis intersect at an appropriate distance from a lens of the camera (or other point of reference on the handle). For example, in embodiments where the intersection between the first optical axis and the first catheter axis is 110 mm from a lens of the camera and where the DoF is at least 60 mm, the accessory device may be used with catheters having an insertable length within the range from 110 mm +/- 30 mm, i.e. from 80 mm to 140 mm, whereby the tip of said catheters will all be within focus of the camera (here, neglecting minor deviations occurring from the choice of refence point). Further, the DoF provides for the surroundings, such as the meatal area and/or meatus, and the tip of the catheter to be in focus simultaneously, such that the images displayed in the display adequately visualize both the tip and the meatal area.

In embodiments, the camera has a DoF between 10 mm and 150 mm, such as between 20 mm and 100 mm, such as between 30 mm and 90 mm, such as between 40 mm and 70 mm, such as 40 mm, 50 mm, 60 mm, or 70 mm.

In embodiments, the camera has a DoF of maximally 10 mm, such as to provide a particularly clear image of a tip end of a catheter, such as where the accessory device is particularly optimised for a certain type of catheter.

In embodiments, the camera has a minimum focus distance of maximally 55 mm (i.e., 55 mm or less), such that the camera can focus on objects (e.g., a tip of a compact catheter) being down to 55 mm from a lens of the camera.

In an embodiment, the accessory device further comprises a light source. In embodiments, the light source is arranged in the handle. In embodiments, the light source is attachable to the handle and/or to the monitor. In embodiments, the light source is arranged in the monitor. In embodiments, the light source is connected to a power unit (e.g., battery) of the monitor, or to a power unit (e.g., battery) of the handle. In embodiments, the light source comprises one or more LED's (diodes), such as a plurality of LED's, such as a plurality of white LED's.

In an embodiment, the light source is arranged circularly about the camera, such as circularly about a lens of the camera. Thereby, the light source can illuminate objects arranged along the first optical axis of the camera. In embodiments, a plurality of LED's is arranged circularly about the camera. Thus, the plurality of LED's may be arranged circularly about the first optical axis the camera.

In an embodiment, the light source is configured to generate a light beam being substantially parallel to the first optical axis of the camera. In embodiments, the light beam is substantially coinciding with the first optical axis of the camera. In embodiments, the light beam is a component light beam comprising a plurality of element light beams as generated by a plurality of individual LED's. In embodiments, the light beam is convergent towards an intersection between the first optical axis and the first catheter axis. In embodiments, the light beam is divergent. In embodiments, the light beam is substantially parallel to and coinciding with the first optical axis of the camera. In embodiments, a parallel, convergent or divergent light beam may be formed by adequately arranging and angling the plurality of LED's about the first optical axis.

By providing a light beam configured to illuminate a tip of a catheter coupled to the handle and in particular to illuminate the surrounding area, such as the meatal area, the benefits of using an accessory device as disclosed is further enhanced.

In an embodiment, the monitor comprises a strap for securing the monitor to an object, such as a leg of a user. Thereby, the monitor may be supported by the object, such that the user has his/her hands free for manipulating the handle with the catheter. In embodiments, the strap is elastic and flexible, such that the strap may be comfortably arranged about a leg of the user. In embodiments, the strap comprises fastening means, such as a buckle or a hook-and-loop fastener for securing the strap tightly to the object. In embodiments, the strap is attached to a back of the monitor, such that the display, being arranged on a front of the monitor, is visible to the user. In embodiments, the strap is attached to a hinge element of the monitor, such that the user may rotate/tilt the monitor relative to the strap and thus the object/leg, such as to provide a comfortable viewing angle for the user. In embodiments, the back of the monitor comprises a protrusion, e.g., a coupling mechanism as discussed below, for providing a natural tilt of the monitor when secured to the object/leg.

In an embodiment, the monitor comprises a coupling mechanism for releasably coupling the handle to the monitor. Thereby, the handle may easily be stowed away, e.g., for storing and transport. In embodiments, the coupling mechanism is a receptacle or a protrusion. In embodiments, the coupling mechanism is a protrusion engageable with the attachment means of the handle, such that the handle, via the attachment means, may be coupled to the monitor. For example, the protrusion may have an outer shape corresponding to an inner shape of the attachment means, such that the attachment means, and thus the handle, may be coupled to the monitor in the same way as a catheter is coupled to the attachment means.

In embodiments, the monitor comprises a recess or groove arranged between the front and the back of the monitor, the recess or groove extending circumferentially about the monitor, thereby forming a cable drum for storing the strap and/or cable connecting the monitor and the camera of the handle.

In an embodiment, the monitor is in communication with the camera through a wired connection. In other words, in an embodiment, a cable connects the monitor and the camera, and optionally a light source, of the handle. The cable is configured to power the camera, and optionally a light source, and to transmit image data from the camera to the monitor. Thereby, the accessory device relies on a physical/wired (as opposed to a wireless) connection between the monitor and the camera. Thereby, the accessory device, such as the camera and/or the monitor, is protected from wireless interruptions and/or unauthorized hacking. Thus, the wired connection provides for the user to rest assured that the images as captured by the camera are not intercepted by an (unauthorized) third-party/man-in-the-middle.

In embodiments, the handle comprises a first interface for receiving a cable, and the monitor comprises a second interface for receiving the cable. In embodiments, the cable is releasably couplable to the handle and/or the monitor, such that the handle, cable and monitor may be disconnected from each other in the first and second interfaces, such as to ease storing. In embodiments, the cable is mounted to the first and second interfaces, such as to avoid unintentional disconnection and to avoid the need for assembling the accessory device before use.

According to an alternative embodiment, the monitor is in communication with the camera through a wireless connection. According to such embodiment, the handle (e.g., interface thereof) and the monitor (e.g., interface thereof) each comprises an antenna and a wireless transceiver for exchanging data, in particular image data indicative of images as captured by the camera. The transceiver of the handle provides for wireless pairing with the transceiver of the monitor and vice versa. In embodiments, the handle is configured to transmit a signal indicative of an image as captured by the camera to the monitor. The wireless transceiver may be a Bluetooth transceiver, i.e. the wireless transceiver may be configured for wireless communication according to a Bluetooth protocol, e.g., Bluetooth Low Energy, Bluetooth 4.0, or Bluetooth 5.

In a second aspect, a catheter system for assisting insertion of a catheter is disclosed. The catheter system comprises an accessory device and a catheter comprising an insertable portion and a connector, the insertable portion having a proximal end. The accessory device comprises a handle comprising an attachment means for releasably coupling the catheter to the handle, and a camera for capturing one or more images, and a monitor in communication with the camera, the monitor comprising a display configured to display the one or more images.

In embodiments, the catheter is a urinary catheter, such as an intermittent urinary catheter, such as an intermittent urinary catheter having an insertable length between 55 mm and 200 mm, such as between 80 mm and 140 mm. In embodiments, the catheter is an anal catheter for anal irrigation, such as an anal catheter having an external diameter between 8 mm and 16 mm, such as 10 mm, and a length between 70 mm and 200 mm.

The catheter system incorporates an accessory device according to the first aspect, and it is appreciated that embodiments and related benefits thereof are applicable to the catheter system as disclosed.

In an embodiment, the camera is configured to focus on the proximal end of the catheter. Thereby, the proximal end (tip) of the catheter as coupled to the attachment means, and thus the handle, is in focus in the image(s) sent to the monitor and displayed on the display thereof.

In a third aspect, a method of using a catheter system is disclosed. The method comprises optionally decoupling the handle from the monitor, coupling the catheter to the handle, observing the images, as captured by the camera, on the display of the monitor to locate the meatal area and the meatus and to guide the catheter into close proximity thereof. In embodiments, the method further comprises inserting the catheter into the meatus.

In embodiments, the handle is coupled to (a back of) the monitor, such as in a transport configuration, and as such, the method may comprise the optional step of decoupling the handle from the monitor. In embodiments, coupling the catheter to the handle comprises coupling the connector of the catheter to the attachment means according to previous embodiments, such as coupling the connector to the attachment means in a sliding motion, wherein the connector becomes frictionally fixated to the attachment means. In embodiments, observing the images on the display of the monitor is provided by means of the camera of the handle capturing images, in particular images of the tip/proximal end of the catheter, which are transmitted to the display. By observing the images of the display, the images thus visualizing the tip of the catheter and vicinity thereof (e.g., the meatal area), the user may manipulate/guide the handle such that the meatal area is within field of view of the camera. Thereby, from the images, the user may locate the meatal area and the meatus, and further, by manipulating/guiding the handle appropriately, guide/bring the catheter into close proximity of the meatus, such that the user may insert the catheter into the meatus. In embodiments, method further comprises decoupling the catheter from the handle, such as prior to micturition/voiding or after micturition/voiding. In embodiments, the catheter is decoupled from the handle according to previous embodiments, such as through a sliding motion, wherein the user provides a force overcoming the friction between the connector of the catheter and the attachment means.

**Detailed description of the drawings**

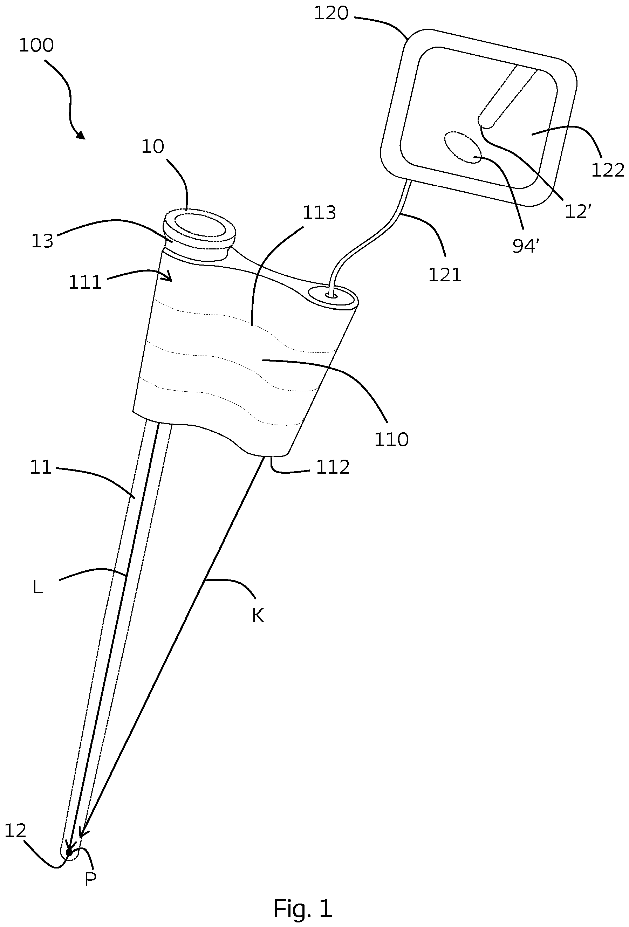

Fig. 1 illustrates a perspective view of an accessory device 100 coupled to a catheter 10. The accessory device 100 comprises a handle 110 and a monitor 120. The handle 110 comprises an attachment means 111, for releasably coupling a catheter 10 to the handle, and a camera 112 for capturing one or more images. The camera 112 and the monitor 120 are connected through a wired connection by means of a cable 121. The body 113 of the handle 110 may have a curved shape, such as to provide an ergonomically grip for the user's hand.

In the illustrated embodiment, a catheter 10 (dashed) is coupled to the attachment means 111. The attachment means 111 fixates the catheter 10 along a first catheter axis L. The first catheter axis L defines the longitudinal direction of the catheter 10. The first catheter axis L may be defined by appropriately engineering the attachment means 111, such that said attachment means repeatably (i.e., every time) fixates a catheter 10 along the same axis, namely the first catheter axis L.

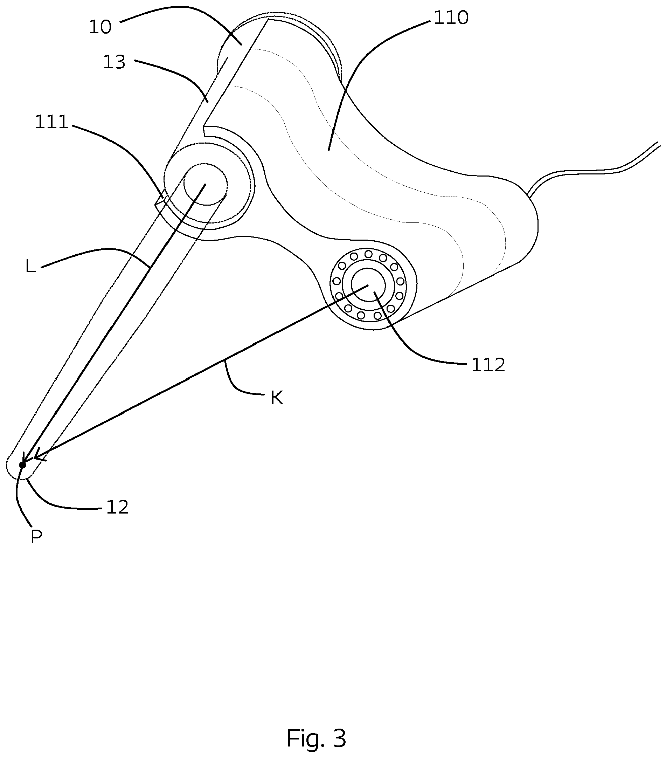

The catheter 10 according to the illustrated embodiment comprises an insertable portion 11, having a proximal end forming a tip 12 of the catheter 10, and a connector 13 arranged distal to the insertable portion 11. According to the illustrated embodiment, the catheter 10 is coupled to the attachment means 111 of the handle 10 through the connector 13 (see also Fig. 3). Thereby, the insertable portion 11 remains sterile and extends into free space, along the first catheter axis L.

The camera 112 is arranged in the handle 110, thereby being fixated relative to the handle 110 and the attachment means 112. The camera 112 comprises a first optical axis K defining a viewing direction of the camera. In other words, the camera 112 is capable of capturing one or more images of objects intersecting the first optical axis K, and surroundings thereof, depending on the field of view. The attachment means 111 and the camera 112 are arranged in the handle and separated by a certain distance, e.g., by 10 mm to 30 mm, such as 20 mm. Further, the attachment means 111 and the camera 112 are angled relative to each other, so as to make the first catheter axis L intersect the first optical axis K at an intersection point P. In other words, the handle 110 is engineered so as to make the first catheter axis L intersect the first optical axis K. In the illustrated embodiment, the handle 110 is engineered such that the intersection point P aligns with the tip 12 of the catheter 10. Thereby, the tip 12 will be in the centre of the images as captured by the camera 112, and consequently in the centre of the display 122 of the monitor 120.

The monitor 120 is connected to the handle 110, in particular to the camera 112 of said handle, by a cable 121. In alternative embodiments, the monitor 120 and the camera 112 are wirelessly connected. The monitor 120 comprises a display 122 configured to display one or more images as captured by the camera 112. The one or more images are transmitted from the camera 112 to the monitor 120 and subsequently displayed on the display 122. In a preferred embodiment, the camera 112 captures a plurality of images consecutively to form a video, which is transmitted to the display 122 without delay, so as to form a live video/footage of the objects within the field of view of the camera 112. In the illustrated embodiment, the tip 12' of the catheter 10 is displayed along with meatus 94', corresponding to the objects within the field of view of the camera 112.

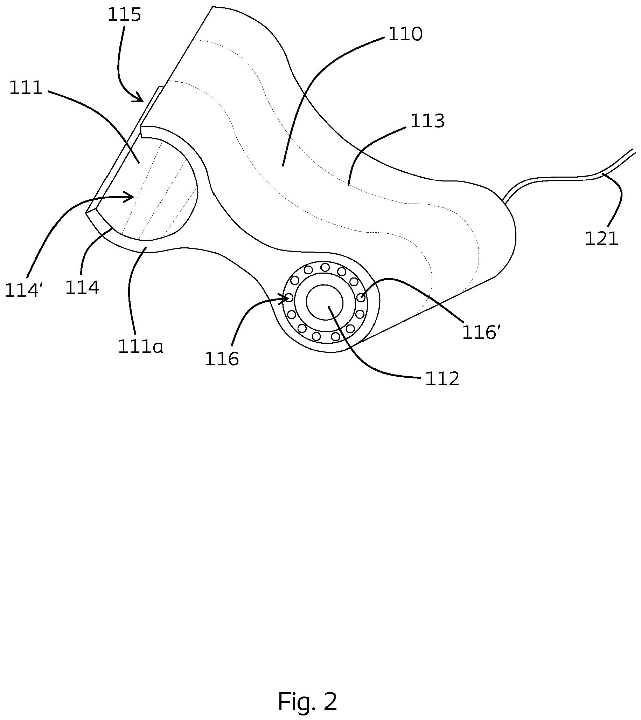

Fig. 2 illustrates a perspective view of an embodiment of the handle 110 of the accessory device 100. The handle 110 comprises an attachment means 111 and a camera 112 arranged in the handle 110. The body 113 of the handle 110 may have a curved shape in a direction from the attachment means 111 to the camera 112, such as to provide an ergonomically grip for the user's hand. In the illustrated embodiment, the curved shape is such that there is a thinner portion between the (portion of the handle 110 holding the) camera 112 and the attachment means 111. The user may prefer to grip the handle 110 with his/her thumb and index finger and this may be made easier by providing a thinner portion between the portion holding the camera and the attachment means.

The attachment means 111 according to the illustrated embodiment comprises a cylindrical portion 114 having a slit 115 for easy insertion of a catheter into the attachment means 111. The cylindrical portion 114 may have an inner conical curvature 114' (illustrated by dotted lines) narrowing towards a proximal end 111a of the attachment means 111, such as to provide fixation of a catheter through a sliding motion, wherein the user slides a connector of a catheter along the first catheter axis until the friction between the connector and the inner conical curvature 114' of the cylindrical portion 114 causes the connector, and as such the catheter, to be (releasably) fixated in/coupled to the attachment means 111. In embodiments, parts of the cylindrical portion 114 is resilient and/or flexible, e.g., supported by a spring or an elastic material, such as to provide a firm grip of a catheter (e.g., connector thereof).

A light source 116 is provided circularly about the camera 112. According to the illustrated embodiment, the light source 116 comprises a plurality of LED's 116' arranged circularly about the camera, such as circularly about the first optical axis of the camera 112. The light source 116 may be powered by a power unit of the handle 110 and/or the monitor, such as powered through the cable 121 extending from the handle 110 to the monitor (not shown). The light source 116 is configured to generate a light beam being substantially parallel to the first optical axis of the camera 112, such that images captured by the camera 112 are illuminated by means of the light source 116.

Fig. 3 illustrates the embodiment of the handle 110 according to Fig. 2, wherein a catheter 10 (dashed) is coupled to the attachment means 111. In particular, the connector 13 of the catheter 10 is coupled to the attachment means 111, as described in relation to Fig. 2. The catheter 10 is fixated by the attachment means 111, the former thereby extending along the first catheter axis L. Thus, a longitudinal direction of the catheter 10 coincides with the first catheter axis L. Further, the first optical axis K of the camera 112 extends in such a way that said first optical axis K intersects the first catheter axis L in an intersection point P (here, coinciding with the tip 12 of the catheter 10). Thereby, the tip 12 of the catheter 10 is ensured to be within the field of view of the camera 112, such that said camera 112 may capture images (e.g., to form a video) of the tip 12 and the vicinity thereof, such as the meatal area, when the handle 110 brings the catheter 10 into proximity with such meatal area.

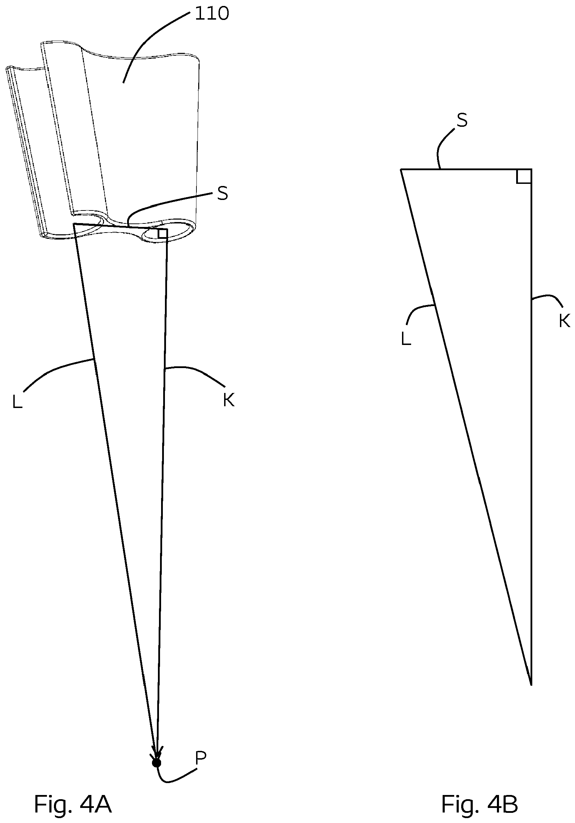

Fig. 4A illustrates an embodiment of a handle 110, wherein the first catheter axis L and the first optical axis K are indicated by vectors/arrows. The first catheter axis L and the first optical axis K intersect at the intersection point P. The first optical axis K and the first catheter axis L are initially (i.e., in the handle 110) separated by a separation S. In order to describe the situation trigonometrically, the first optical axis K and the separation S according to the embodiment form a right angle.

Fig. 4B illustrates a trigonometric representation of the relation between the first optical axis K and the first catheter axis L as defined by the structural features of the handle 110. The trigonometric representation is based on the embodiment of the handle 110 as illustrated in Fig. 4A.

In order to accommodate a large range of different catheters, in particular catheters having different (insertable) lengths, and to have the meatal area and the tip of the catheter in focus simultaneously, the camera may be engineered to have a certain depth of field (DoF). By having a suitable DoF, the exact intersection point P between the optical axis K and the catheter axis L does not limit the functionality of the accessory device. Rather, the DoF provides for having shorter or longer catheters to be in focus as well, along with providing for having the meatal area in focus simultaneously, when the tip of the catheter is in close proximity thereof.

The below deviation should not be considered limiting to the scope of the invention. Instead, the deviation intends to clarify and highlight how appropriately choosing a DoF allows for using the accessory device according to embodiments of the disclosure with a large range of different catheters. Thus, whereas assumptions provided herein are based on the trigonometric representation according to Fig. 4B, it is appreciated that different representations may be more suitable for an accessory device according to embodiments of the disclosure.

Generally, a needed DoF may be calculated as

$$DoF = K_{max} - K_{min}$$

where $K_{max}$ is the maximum needed focus distance (distance from camera lens to object to be in focus) and $K_{min}$ is the minimum needed focus distance. $K_{max}$ and $K_{min}$ may be calculated by applying trigonometric functions to the trigonometric representation of Fig. 4B:

$$K_{max} = \frac{S}{\tan \sin^{-1} \dfrac{S}{L_{max}}}$$

$$K_{min} = \frac{S}{\tan \sin^{-1} \dfrac{S}{L_{min}}}$$

where a right-angled triangle is assumed (see Fig. 4B), and where S is the separation between the optical axis K and the catheter axis L in the handle (corresponding to the shorter side of the triangle), $L_{max}$ is the maximum

insertable length of a catheter to be used with the present accessory device and $L_{min}$ is the minimum insertable length of a catheter to be used with the present accessory device.

For example, assuming a separation S of 20 mm, and assuming the accessory device being configured to accommodate catheters having insertable lengths in the range from 80 mm to 140 mm, the needed DoF is 61 mm (preferably larger to account for deviations from the applied trigonometric representation). Assuming a separation S of 30 mm, the corresponding DoF is 63 mm, and for S=10 mm, the corresponding DoF is 60 mm.

Thus, the above deviation reveals that the accessory device may effectively be used with catheters having insertable portions having lengths in the range from 80 mm to 140 mm, provided that the DoF of the camera has been engineered to be approximately 60 mm.

The DoF of the camera may be engineered by choosing appropriate parameters/optics of the camera, e.g., by choosing appropriate values for the focal length, aperture size and acceptable circle of confusion size.

By engineering a certain DoF allows for the camera to have the tip of the catheter in focus irrespective of the insertable length of the catheter (within the limits as discussed). Therefore, the first optical axis of the camera and the first catheter axis need not to intersect exactly at the tip of the catheter used to have said tip in focus.

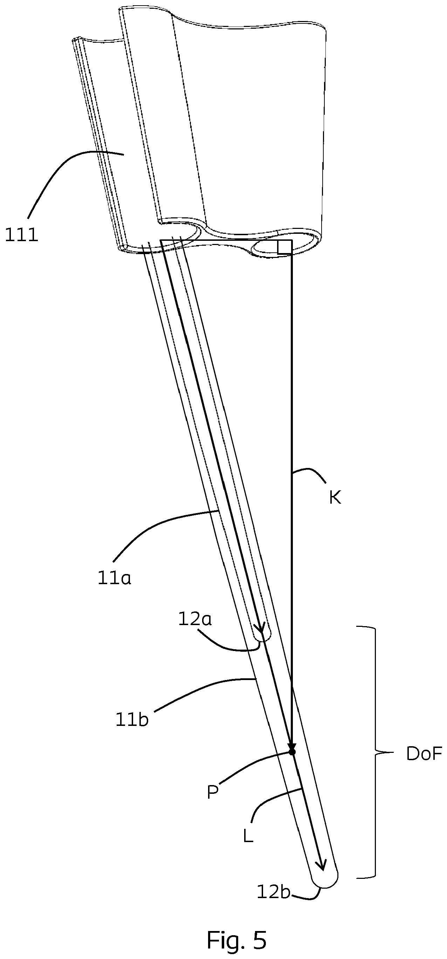

Fig. 5 illustrates a short insertable portion 11a (dashed line) of a catheter coupled to the attachment means 111 layered with a long insertable portion 11b (solid line) (connectors not shown). The first catheter axes L of each of the two insertable portions 11a,11b are coinciding due to the attachment means 111 fixating a catheter along the same first catheter axis L. The first optical axis K intersects the first catheter axis L at an intersection point P arranged at a point along the first catheter axis L between a tip 12a of the short insertable portion 11a and a tip 12b of the long insertable portion 11b. The camera has a DoF as indicated, which covers each of the tips 12a,12b and the intersection point P. Thereby, both the tip 12a of the short insertable portion 11a and the tip 12b of the long insertable portion 11b are within focus of the camera 112. Thereby, the images generated by the camera are in focus for both catheters, such that the user may choose to use either catheter without risking having a bad image quality caused by the tip of his/her preferred catheter being out of focus. Further, the DoF allows for having the meatal area within focus as well, when the catheter coupled to the handle is brought into close proximity thereof.

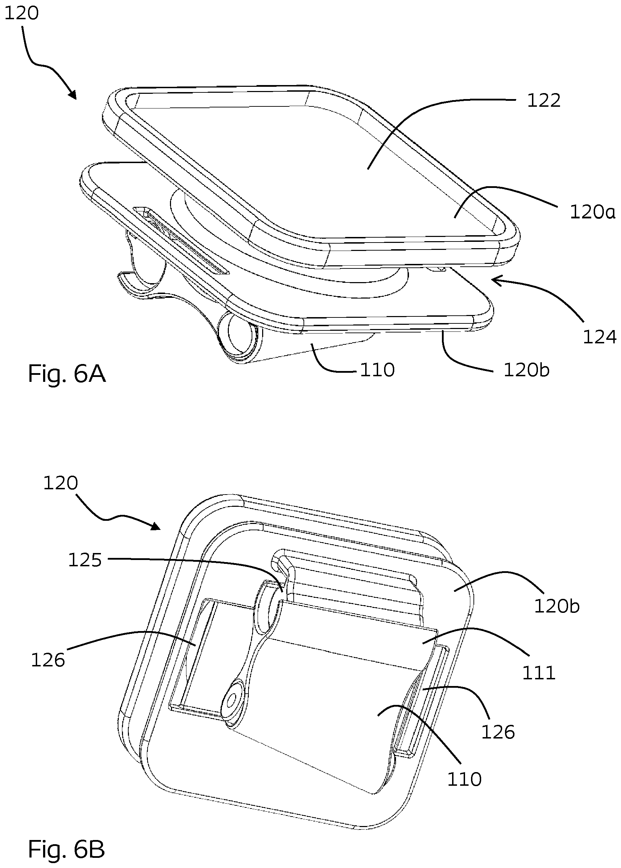

Fig. 6A illustrates a perspective view of a monitor 120 of an accessory device according to an embodiment of the disclosure. The monitor 120 comprises a front 120a and a back 120b. The front 120a of the monitor 120 comprises a display 122 configured to display images captured by the camera of the accessory device. The display 122 may be a commercially available display, such as an LED display or an LCD display.

The monitor 120 may comprise a power unit, such as a battery, for powering the display and/or the camera of the handle, and optionally a light source. The monitor 120 may comprise a charging port for charging the battery. The monitor 120 may comprise a processor for processing images as captured by a camera of the accessory device and/or for handling the displaying of the images.

A groove 124 may be arranged circumferentially about the monitor 120, such as between the front 120a and the back 120b, such as to form a cable drum for storing a strap and/or cable during storage and/or transport.

A monitor 120 according to embodiments of the disclosure may have dimensions (width x length x thickness) in the range from (4 cm to 8 cm)x(4 cm to 8 cm)x(0.5 cm to 3 cm). The cable may have a length between 20 cm and 100 cm.

The handle 110 (cable not shown) of the accessory device is coupled to the back 120b of the monitor 120 by means of a coupling mechanism, as will be explained in relation to Fig. 6B below.

Fig. 6B illustrates a perspective view of a monitor 120 of an accessory device according to an embodiment of the disclosure. The back 120b of the monitor 120 comprises a coupling mechanism 125, here embodied as a protrusion, for releasably coupling the handle 110 to the monitor 120. The coupling mechanism 125 may be correspond to the shape of a connector of a catheter, such that the handle 110 may be coupled to the monitor 120 according to the same principle as coupling a catheter to the attachment means 111. Thus, the coupling mechanism 125 may be substantially cylindrical or conical having an outer curvature corresponding to an inner curvature of the attachment means 111 of the handle 110. Thereby, the monitor 120 and the handle 110 may be easily coupled to provide a compact accessory device 100, making it easy to bring the accessory device 100 along, and to protect the camera during storage/transport.

The back 120b of the monitor 120 may comprise two parallel slits 126 configured to receive a strap for securing the monitor 120 to an object, e.g., a leg of the user. Alternatively, the back 120b of the monitor may comprise an adhesive, such as a reusable adhesive, for adhering the monitor to a leg of the user.

Where the monitor 120 is secured to an object, e.g., a leg of the user, the coupling mechanism 125 (with the handle detached) may provide a support causing the monitor 120 to be tilted, thereby providing a comfortable viewing angle for the user during the catherization procedure.

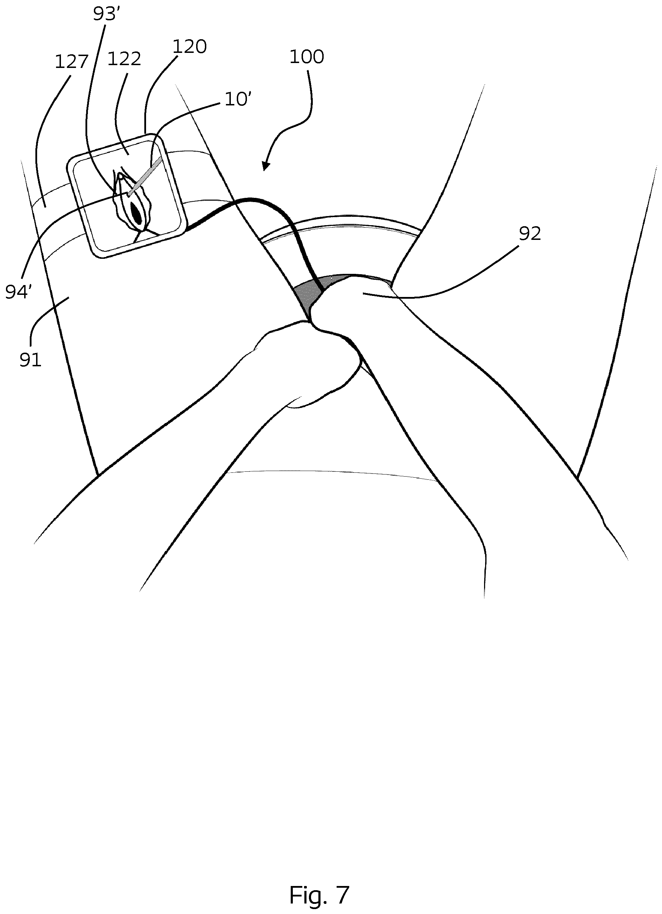

Fig. 7 illustrates a use situation of the accessory device 100 as described herein. The monitor 120 is secured to a leg 91 of the user by means of a strap 127. The handle (not visible) is manipulated by the user's hands 92. On the display 122 of the monitor 120, the user can see her meatal area 93' and meatus 94' (e.g., external urethral orifice) by means of the images/video recorded by the camera (not visible) of the handle. A catheter 10' (as visible on the display 121 of the monitor) is coupled to the attachment means of the handle, such that the user can guide the tip of the catheter into meatus by looking at the display 122. Thereby, the user may avoid contaminating the sterile insertable portion of the catheter, and as such, may avoid contracting a urinary tract infection caused by such contamination. When the catheter has been inserted, e.g., prior to, or after, micturition/voiding, the user may decouple the catheter from the attachment means of the handle and proceed as he/she prefers.

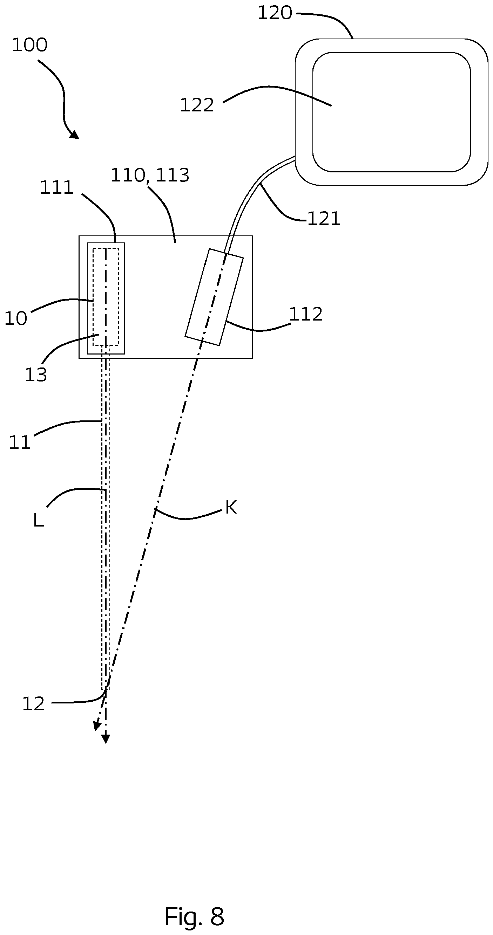

Fig. 8 illustrates a schematic view of an accessory device 100 as described herein. The accessory device 100 comprises a handle 110 comprising an attachment means 111 for releasably coupling a catheter 10 (dashed) to the handle, and a camera 112 for capturing one or more images. Further, the accessory device 100 comprises a monitor 120 in communication with the camera 112, such as through a wired connection by means of a cable 121. Further, the monitor comprises a display 122 configured to display the one or more images.

The camera 112 and the attachment means 111 may be fixated in a body 113 of the handle 110, e.g., a body having dimensions providing for a handheld handle 110. The attachment means 111 may be configured to releasably coupling a connector 13 of the catheter 10 to the handle 110, such as to provide for the insertable portion 11 of the catheter 10 to extend into free space. Preferably, the attachment means 111 provides for fixating the catheter 10 along a first catheter axis L (dashed-dotted), and the camera 112 is arranged in handle 110 such as to define a first optical axis K (dashed-dotted) defining a viewing direction of the camera 112. Preferably, the first optical axis K intersects the first catheter axis, as illustrated. Thereby, the camera 112 is configured to capture images of at least parts of the catheter 10, such as specifically a tip 12 of the catheter. Preferably, the camera 112 provides for having the tip 12 and the vicinity of the tip (such as a meatal area) in focus simultaneously.

Although particular features have been shown and described, it will be understood that they are not intended to limit the claimed invention, and it will be made obvious to those skilled in the art that various changes and modifications may be made without departing from the spirit and scope of the claimed invention. The specification and drawings are, accordingly, to be regarded in an illustrative rather than restrictive sense. The claimed invention is intended to cover all alternatives, modifications, and equivalents.

Embodiments of the present disclosure are set out in the following items.

1. An accessory device for assisting insertion of a catheter, wherein the accessory device comprises

- a handle comprising an attachment means for releasably coupling a catheter to the handle, and a camera for capturing one or more images; and
- a monitor in communication with the camera, the monitor comprising a display configured to display the one or more images.

2. The accessory device according to item 1, wherein the attachment means comprises a clip for releasably coupling a connector of a catheter to the handle.

3. The accessory device according to item 2, wherein the clip is configured for fixating a connector of a catheter by means of friction.

4. The accessory device according to item 3, wherein the clip comprises a conical inner shape configured to receive and fixate a connector of a catheter.

5. The accessory device according to any one of items 1-4, wherein the attachment means is configured for fixating a catheter along a first catheter axis.

6. The accessory device according to any one of items 1-5, wherein the camera is fixated in the handle relative to the attachment means.

7. The accessory device according to any one of items 1-6, wherein the camera comprises a first optical axis defining a viewing direction.

8. The accessory device according to item 7 as dependent on item 5, wherein the first optical axis intersects the first catheter axis.

9. The accessory device according to item 7 as dependent on item 5, wherein the first optical axis intersects the first catheter axis at a distance between 55 mm and 200 mm from a lens of the camera.

10. The accessory device according to any one of items 1-9, wherein the camera has a depth of field of at least 60 mm.

11. The accessory device according to any one of items 1-10, wherein the accessory device further comprises a light source.

12. The accessory device according to item 11, wherein the light source is arranged circularly about the camera.

13. The accessory device according to any one of items 11-12 as dependent on item 7, wherein the light source is configured to generate a light beam being substantially parallel to the first optical axis of the camera.

14. The accessory device according to any one of items 1-13, wherein the monitor comprises a strap for securing the monitor to an object.

15. The accessory device according to any one of items 1-14, wherein the monitor comprises a coupling mechanism for releasably coupling the handle to the monitor.

16. The accessory device according to any one of items 1-15, wherein the monitor is in communication with the camera through a wired connection.

17. The accessory device according to any one of items 1-16, wherein the accessory device has a liquid ingress protection rating of at least IPX4 according to the IEC standard 60529.

18. A catheter system for assisting insertion of a catheter, the catheter system comprising an accessory device and a catheter comprising an insertable portion and a connector, the insertable portion having a proximal end, wherein the accessory device comprises

- a handle comprising an attachment means for releasably coupling the catheter to the handle, and a camera for capturing one or more images, and
- a monitor in communication with the camera, the monitor comprising a display configured to display the one or more images.

19. The catheter system according to item 18, wherein the camera is configured to focus on the proximal end of the catheter.

20. The catheter system according to any one of items 18-19, wherein the catheter is an intermittent urinary catheter.

21. A method of using a catheter system as in items 18-20, wherein the method comprises

- optionally decoupling the handle from the monitor,
- coupling the catheter to the handle,
- observing images, as captured by the camera, on the display of the monitor to locate the meatal area and the meatus, and
- guiding the catheter into close proximity of the meatus.

**Claims**

1. An accessory device (100) for assisting insertion of a catheter (10) into a meatus, wherein the accessory device (100) comprises

- a handle (110) comprising an attachment means (111) for releasably coupling a catheter (10) to the handle and for fixating the catheter relative to the handle, and a camera (112) for capturing one or more images, the camera being fixated in the handle relative to the attachment means; and
- a monitor (120) in communication with the camera (112), the monitor (120) comprising a display (122) configured to display the one or more images whereby movements caused by a user handling the handle (110) are reflected in the one or more images displayed in the display.

2. The accessory device according to claim 1, wherein the attachment means comprises a clip for releasably coupling a connector (13) of a catheter to the handle.

3. The accessory device according to claim 2, wherein the clip is configured for fixating a connector (13) of a catheter by means of friction.

4. The accessory device according to claim 3, wherein the clip comprises a conical inner shape configured to receive and fixate a connector of a catheter.

5. The accessory device according to any of claims 1-4, wherein a viewing direction of the camera (112) points towards a tip of a catheter coupled to the attachment means (111).

6. The accessory device according to any of claims 1-5, wherein the camera (112) and a catheter coupled to the attachment means (111) are configured to move in unison when the user manipulates the handle (110).

7. The accessory device according to any one of claims 1-6, wherein the attachment means is configured for fixating a catheter along a first catheter axis (L).

8. The accessory device according to any one of claims 1-7, wherein the camera comprises a first optical axis (K) defining a viewing direction.

9. The accessory device according to claim 8 as dependent on claim 7, wherein the first optical axis (K) intersects the first catheter axis (L).

10. The accessory device according to claim 9, wherein the first optical axis (K) intersects the first catheter axis (L) at a distance between 55 mm and 200 mm from a lens of the camera.

11. The accessory device according to any one of claims 1-10, wherein the camera has a depth of field of at least 60 mm.

12. The accessory device according to any one of claims 1-11, wherein the monitor is configured to mirror the one or more images in the display.

13. A catheter system for assisting insertion of a catheter (10) into a meatus, the catheter system comprising an accessory device (100) and a catheter (10), the catheter (10) comprising an insertable portion (11) and a connector (13), the insertable portion (10) having a proximal end, wherein the accessory device comprises

- a handle (110) comprising an attachment means (111) for releasably coupling the catheter (10) to the handle and for fixating the catheter relative to the handle, and a camera (112) for capturing one or more images, the camera being fixated in the handle relative to the attachment means; and
- a monitor (120) in communication with the camera (112), the monitor (120) comprising a display (122) configured to display the one or more images whereby movements caused by a user handling the handle (110) are reflected in the one or more images displayed in the display.

14. The catheter system according to claim 13, wherein the camera (112) is configured to focus on the proximal end of the catheter (10).

15. The catheter system according to any one of claims 13-14, wherein the catheter (10) is an intermittent urinary catheter or an anal catheter.

Fig. 1

Fig. 2

Fig. 3

Fig. 4A

Fig. 4B

Fig. 5

Fig. 6A

Fig. 6B

Fig. 7

Fig. 8